# EUROPÄISCHE PATENTSCHRIFT

(11) **EP 3 092 966 B1**
(45) Veröffentlichungstag und Bekanntmachung des Hinweises auf die Patenterteilung: **20.01.2021**
(21) Anmeldenummer: 15167061.9
(22) Anmeldetag: 11.05.2015
(51) Int. Cl.: A61B 18/04, A61B 18/14, A61B 18/00, A61B 17/00

(54) **ELEKTROCHIRURGISCHES INSTRUMENT**
ELECTROSURGICAL INSTRUMENT
INSTRUMENT ÉLECTROCHIRURGICAL

(43) Veröffentlichungstag der Anmeldung: 16.11.2016
(73) Patentinhaber: Erbe Elektromedizin GmbH, 72072 Tübingen (DE)
(72) Erfinder: Kirstgen, Udo, 72108 Rottenburg (DE); Staneker, Peter, 72829 Engstingen (DE); Rombach, Thorsten, 72810 Gomaringen (DE)
(74) Vertreter: Bohnenberger, Johannes

(56) Entgegenhaltungen:
- EP-A1- 0 634 139
- DE-C1- 4 414 810
- US-A1- 2008 051 802
- US-A1- 2008 200 761
- US-A1- 2010 211 076
- US-A1- 2015 073 341
- US-A1- 2015 119 851

## Beschreibung

Die Erfindung bezieht sich auf ein elektrochirurgisches Instrument, insbesondere für die Argonplasma-Koagulation, mit den Merkmalen des Oberbegriffs des Anspruchs 1. Ein solches Instrument ist beispielsweise aus US 2009/0125023 A1 bekannt. Dokumente US2008051802 A1, US2015073341 A1, US2012029506 A1, US2013261536 A1 und EP1595507 A2 offenbaren weitere relevante Vorrichtungen aus dem Stand der Technik. Die Erfindung betrifft ferner ein Gerät mit einem solchen Instrument.

Elektrochirurgische Instrumente der eingangs genannten Art werden zum Schneiden oder zum Koagulieren von Gewebe mit hochfrequentem Wechselstrom eingesetzt. Die Argonplasma-Koagulation ist eine spezielle Anwendungsform der Elektrochirurgie, bei der HF-Strom kontaktlos über ionisiertes Argongas übertragen wird.

Die Wirkung des Energieeintrages erfolgt bei dem eingangs genannten bekannten Instrument dadurch, dass die exponierte Länge der Elektrode geändert wird. Dazu ist ein axial beweglicher Außenschaft vorgesehen, der die Elektrode isolierend umgibt und entlang der Elektrode verschoben werden kann, um diese je nach Bedarf zu exponieren.

Eine zentrale Anforderung an derartige Instrumente ist die Möglichkeit der Ein-Hand-Bedienung. Dabei soll sich die Position des Instruments im OP-Feld möglichst nicht ändern. Dies bedeutet, dass die Griffhaltung bei der Bedienung des Instruments möglichst beibehalten werden soll, auch wenn der Außenschaft verschoben wird. Bei dem gattungsgemäßen Instrument wird dies durch ein Drehrad erreicht, das zentral im Handgriff des Instrumentes angeordnet ist und mit dem Zeigefinger betätigt werden kann. Das Drehrad treibt den Außenschaft an, der dadurch entlang der Elektrode axial verschoben werden kann.

Der Außenschaft des bekannten Instrumentes ist starr und erstreckt sich gerade in distaler Richtung. In bestimmten Anwendungsfällen ist eine abgewinkelte Schaftstellung wünschenswert. Hierfür existieren zwar APC Applikatoren mit flexiblen Spitzen. Die Spitzen müssen aber vor der Anwendung manuell in die gewünschte Winkellage gebogen werden. Eine Änderung der Winkellage im Gebrauch ist nicht möglich.

Der Erfindung liegt die Aufgabe zugrunde, das eingangs genannte elektrochirurgische Instrument dahingehend zu verbessern, dass eine Einstellung der Winkellage durch eine Betätigung im Bereich des Handgriffes, insbesondere auch während der Anwendung des Instrumentes möglich ist. Der Erfindung liegt ferner die Aufgabe zugrunde, ein Gerät mit einem solchen Instrument anzugeben.

Erfindungsgemäß wird diese Aufgabe durch ein elektrochirurgisches Instrument mit den Merkmalen des Anspruchs 1 bzw. durch ein Gerät mit den Merkmalen des Anspruchs : 14 gelöst.

Die Offenbarung umfasst demnach ein elektrochirurgisches Instrument, insbesondere für die Argonplasma-Koagulation, mit einem Handgriff, einem Außenschaft, der eine Elektrode und/oder einen Innenschaft umgibt und im Handgriff gehalten ist, und einem Betätigungsmechanismus am Handgriff zur Bewegung des Außenschafts in axialer Richtung relativ zur Elektrode/zum Innenschaft. Der Außenschaft ist mit dem Innenschaft/der Elektrode in einem distalen Endbereich des Außenschafts mechanisch verbunden. Der Innenschaft/Die Elektrode ist durch die Bewegung des Außenschafts relativ zum Innenschaft/zur Elektrode abwinkelbar.

Das Instrument hat einen einfachen Aufbau, da mit einer geringen Anzahl von Bauteilen die angestrebte Funktionalität, nämlich die Einstellung eines Winkels im Bereich der Schaftspitze durch eine Betätigung des Handgriffes erreicht wird. Daher eignet sich das Instrument als kostengünstiges und damit wirtschaftliches Einwegprodukt.

Vorzugsweise weist die Elektrode einen ersten, einen zweiten und einen dritten Abschnitt auf und erstreckt sich vom Handgriff aus durch den Außenschaft hindurch. Die Elektrode ist im zweiten Abschnitt flexibel ausgebildet. Der zweite Abschnitt der Elektrode ist bei dieser Ausführung über eine Hülse mit dem Außenschaft axial fixiert. Durch eine axiale Bewegung des Außenschafts wird dieser zusammen mit dem zweiten Abschnitt der Elektrode abgewinkelt und somit aus seiner Position quer zur Längsachse des Schafts ausgelenkt. Der erste Abschnitt der Elektrode ist proximal vom abwinkelbaren Bereich des Schafts angeordnet und ortsfest mit dem Handgriff verbunden und somit mit diesem fixiert. Der erste Abschnitt der Elektrode ist starr. Der dritte Abschnitt der Elektrode ist distal beabstandet vom proximalen Ende des abwinkelbaren Bereich des Schafts und somit distal vom zweiten Abschnitt der Elektrode angeordnet. Alle drei Abschnitte der Elektrode sind einstückig, nahtlos miteinander verbunden.

Die vom Handgriff aus einstellbare Winkelposition des Außenschafts wird bei dieser Ausführungsform dadurch erreicht, dass die Elektrode mit dem Handgriff ortsfest verbunden und somit axial fixiert ist. Zusätzlich ist in einem Bereich, der distal vom proximalen Ende des abwinkelbaren Bereichs beabstandet ist ein erstes Ende der Hülse mit der Elektrode ortsfest verbunden und somit axial fixiert. Das zweite Ende der Hülse, welches proximal des abwinkelbaren Bereichs angeordnet ist, ist mit dem Außenschaft fixiert. Durch die Bewegung des Außenschafts wird der Abstand zwischen der Fixierstelle der Hülse mit der Elektrode und dem Handgriff, konkret dem distalen Ende des Handgriffes, verändert. Wird der Abstand verkürzt, wird der abwinkelbare Bereich des Außenschafts aus dem geraden Ruhezustand ausgelenkt und die Schaftspitze in die gewünschte Position bewegt. Dabei wird ein Winkel zwischen der Schaftspitze und der Längsachse des Handgriffes bzw. dem aus dem Handgriff ragenden Außenschaft eingestellt.

Um die stauchende bzw. streckende Wirkung zu erreichen, ist die Fixierung der Elektrode distal vom proximalen Ende des abwinkelbaren Bereich beabstandet. Damit wird erreicht, dass der abwinkelbare Bereich im Bereich zwischen dem proximalen Ende und der Fixierstelle ausgelenkt wird. Je größer der Abstand zwischen dem proximalen Ende und der Fixierstelle ist, umso länger ist die für die Auslenkung wirksame Strecke. Besonders günstig ist die Fixierung der Elektrode im Bereich des distalen Endes des abwinkelbaren Bereichs, weil damit die gesamte Bereichslänge genutzt wird. Hierbei ist die Elektrode proximal vom abwinkelbaren Bereich starr und distal beabstandet von einem proximalen Ende des abwinkelbaren Bereichs am Außenschaft fixiert.

Es versteht sich allgemein, dass die Elektrode zusätzlich zu der distalen Fixierung am Außenschaft mittels der Hülse auch proximal in Längsrichtung der Elektrode mit dem Handgriff fixiert ist, um die Kräfte abzustützen, die über die distale Fixierung der Elektrode eingeleitet werden.

Unter dem abgewinkelten Zustand wird allgemein ein Zustand verstanden, bei dem der Außenschaft entlang seiner exponierten Gesamtlänge nicht geradlinig ist, so dass die Schaftspitze in eine Richtung weist, die von der Längsachse des aus dem Handgriff ragenden Außenschafts abweicht. Im abgewinkelten Zustand schließen das proximale Ende und das distale Ende des abwinkelbaren Bereichs einen Winkel ein. Im Bereich zwischen dem proximalen und distalen Ende kann der Bereich im abgewinkelten Zustand gekrümmt sein.

Die Größe des Winkels hängt von dem Auslenkungsgrad des Außenschafts im Bereich des abwinkelbaren Bereichs ab, der wiederum durch die Axialbewegung des Außenschafts bestimmt wird.

Durch Ausschieben des Außenschafts wird der Abstand zwischen der Fixierstelle der Hülse mit der Elektrode und dem Handgriff vergrößert, so dass der gekrümmte bzw. abgewinkelte Bereich gestreckt und der Winkel zwischen den beiden Schaftabschnitten verringert bzw. vollständig aufgehoben wird, so dass der Außenschaft in den geraden Ruhezustand zurückkehrt.

Die Elektrode hat damit eine Doppelfunktion. Zum einen dient sie zur Leitung des elektrischen Stroms. Zum anderen hat die Elektrode eine mechanische Funktion, nämlich als Schub- bzw. Zugstange, die die vom Betätigungsmechanismus über den Außenschaft eingeleiteten Zug- bzw. Schubkräfte überträgt und damit die Auslenkung des abwinkelbaren Bereichs bewirkt. Zur sicheren Übertragung der Kräfte ist die Elektrode im Bereich proximal vom abwinkelbaren Bereich starr ausgebildet. Da die Elektrode im zweiten Abschnitt flexibel ist, folgt die Elektrode der Krümmung des abwinkelbaren Bereichs.

Die Doppelfunktion der Elektrode hat den Vorteil, dass auf zusätzliche mechanische Bauteile, wie beispielsweise Zugseile verzichtet wird.

Außerdem ermöglicht die Offenbarung, die Einhandbedienung beizubehalten, so dass das Instrument ergonomisch günstig bedient werden kann.

Weitere bevorzugte Ausführungsformen der Erfindung sind in den Unteransprüchen angegeben.

Vorzugsweise ist die Elektrode am distalen Ende des abwinkelbaren Bereichs fixiert. Dies hat den Vorteil, dass die gesamte Länge des abwinkelbaren Bereichs für die Auslenkung genutzt wird.

Der abwinkelbare Bereich weist vorzugsweise eine flexible, insbesondere längs- und/oder quergeschlitzte, Hülse auf, die die Elektrode umgibt. Die Hülse dient als Scharnier für den abwinkelbaren Bereich. Bei einer besonders bevorzugten Ausführung ist die Elektrode mit der Hülse zur Übertragung von Druck- und Zugkräften verbunden. Dabei werden die durch den Betätigungsmechanismus über den Außenschaft eingeleiteten Kräfte von der Elektrode auf die Hülse übertragen, die aufgrund ihrer flexiblen Eigenschaften im abwinkelbaren Bereich aus der geradlinigen Ruhelage ausgelenkt und gekrümmt wird.

Ein proximales Ende der Hülse kann mit einem ersten starren Außenschaftabschnitt verbunden sein. Dadurch werden die über die Elektrode eingeleiteten Kräfte abgestützt, so dass die Hülse durch die Betätigung des Betätigungsmechanismus ausgelenkt wird.

Vorzugsweise ist das distale Ende der Hülse mit einem zweiten starren Außenschaftabschnitt verbunden, der den dritten Abschnitt der Elektrode aufnimmt, der als Zündelektrode dient. Der abwinkelbare Bereich ist also zwischen den beiden starren Außenschaftabschnitten angeordnet.

Zur Verbesserung der Stabilität des Außenschafts können der erste und/oder der zweite Außenschaftabschnitt jeweils ein Stützrohr aufweisen, das einerseits mit der Hülse und andererseits mit einem Außenschaftrohr aus Kunststoff verbunden ist.

Bei dem Stützrohr kann es sich um ein metallisches Stützrohr handeln, das eine ausreichende Festigkeit zur Aufnahme der im Gebrauch auftretenden Kräfte aufweist. Das Stützrohr ist also einerseits mit der Hülse und andererseits mit einem Schaftrohr verbunden und kann aus Kunststoff sein. Das proximale Schaftrohr führt zum Handgriff. Das distale Schaftrohr bildet den zweiten starren Schaftabschnitt, der beispielsweise in einer keramischen Endhülse enden kann, die mit dem Schaftrohr verbunden ist.

Vorzugsweise weist der abwinkelbare Bereich einen Schlauch, insbesondere einen Schrumpfschlauch auf, der die Außenschaftaußenwand im Bereich des abwinkelbaren Bereichs bildet. Dadurch wird eine gasdichte Ummantelung geschaffen, die zudem die Elektrode nach außen elektrisch isoliert. Der Schlauch ist flexibel, um das Abwinkeln des Bereichs zu ermöglichen.

Bei einer weiteren Ausführungsform ist vorgesehen, dass der Innenschaft im distalen Endbereich einen abwinkelbaren Bereich aufweist und der Innenschaft in diesem Bereich flexibel ausgebildet ist, wobei der abwinkelbare Bereich des Innenschafts durch die Bewegung des Außenschafts relativ zum Innenschaft abwinkelbar ist.

Die Zug- und Druckkräfte zur Abwinklung des Innenschafts werden durch die Relativbewegung zwischen dem Außen- und Innenschaft erzeugt. Der Innenschaft ist ortsfest mit dem Handgriff verbunden.

Vorzugsweise ist der Innenschaft als Elektrode ausgebildet und dient als elektrischer Leiter beispielsweise eines Hochfrequenzstroms für monopolares Koangulieren.

Hierdurch wird erreicht, dass ein abwinkelbarer Innenschaft zur Verfügung gestellt wird der über einen weiteren Freiheitsgrad verfügt und gleichzeitig auf einfache Weise als Elektrode genutzt werden kann, wodurch das separate Vorsehen einer Elektrode obsolet wird.

Diese Ausführung hat den Vorteil, dass Einbauteile zur Verbindung der Elektrode mit dem Außenschaft entfallen. Das Lumen des Innenrohres steht für den Durchfluss von Medien, bspw. zum Absaugen, zur Verfügung.

Andererseits ist denkbar, dass eine Elektrode innerhalb des Innenschafts angeordnet ist. Hierbei umgibt der Innenschaft die Elektrode. Die Elektrode wird beim Abwinkeln des Innenschafts zusammen mit dem Innenschaft mitbewegt. Der Innen- und Außenschaft sind voneinander elektrisch isoliert.

Die Fixierung zwischen Innen- und Außenschaft, vorzugsweise im Bereich der Schaftspitze, kann durch ein Verbindungselement mechanisch vorgenommen werden. Das Verbindungselement ist vorzugsweise als Band, Stift, Lasche oder Steg ausgebildet. Das Verbindungselement ist vorzugsweise flexibel sowie vorzugsweise aus Metall ausgebildet.

Das Verbindungselement ist einerseits mit dem Außenschaft und andererseits mit dem Innenschaft verbunden.

Vorzugsweise ist das Verbindungselement im Bereich des abwinkelbaren Bereichs des Innenschafts und noch bevorzugter distal des abwinkelbaren Bereichs des Innenschafts angeordnet und überbrückt somit den abwinkelbaren Bereich des Innenschafts.

Der abwinkelbare Bereich des Innenschafts kann in dem entsprechenden Wandabschnitt Schlitze aufweisen. Die besondere Anordnung der Schlitze ermöglicht, dass der Innenschaft in axialer Richtung Kräfte aufnimmt und sich quer zur Längsachse des Innenschafts biegen lässt.

Andererseits kann der abwinkelbare Bereich des Innenschafts durch gegenüberliegende Schlitze ausgebildet sein. Diese Schlitze werden vorzugsweise durch ein Laserstrahlschneideverfahren ausgebildet.

Die Schlitze reichen vorzugsweise jeweils über die Achsmitte des Innenschafts, sodass eine Art Knick-Strohhalm Effekt erreicht wird. Die gegenüberliegenden Schlitze sind in diesem Fall versetzt zueinander ausgebildet und überlappen sich im Bereich der jeweiligen Schlitzspitzen.

Denkbar ist, dass sich die jeweiligen Schlitzspitzen nur geringfügig überlappen und daher ein geringer Überlappungsgrad vorliegt. Der Überlappungsgrad kann so gewählt werden, dass die Stabilität des Innenschafts sichergestellt ist und gleichzeitig ein ausreichender Abwinklungswinkel ermöglicht wird.

Vorzugsweise sind die Schlitze in Längsrichtung des Innenschafts mit gleichen Abständen zueinander angeordnet, wodurch ein gleichmäßiges Abwinkeln des Innenschafts ermöglicht wird.

Die Schlitze sind bevorzugt kammartig ausgebildet, wobei durch die Versetzung der Schlitze eine Art Ineinandergreifen der gegenüberliegenden Schlitze ermöglicht wird.

Je größer die Überlappung im Überlappungsbereich ist, desto größer ist der Federweg eines Einzelelements. Größere Überlappung führt daher zu einer geringeren Rückstellkraft.

Der Winkelbereich, in dem der Innenschaft im abwinkelbaren Bereich abwinkelbar ist, ist größer, je mehr Schlitze in das Rohr eingebracht werden. Außerdem vergrößert sich der Winkelbereich bei gleichbleibender Schlitzanzahl, je größer die Schlitzbreite ist.

Der Bereich, indem die Schlitze überlappen, beschreibt eine Federgeometrie, wobei der Innenschaft nicht plastisch verformt wird, sondern die entstehenden Federelemente dafür sorgen, dass eine elastische Verformung des Innenschafts ermöglicht wird.

Die Breite der Verbindungsstege darf nicht zu groß gewählt werden, da die elastische Verformung sonst in eine plastische Verformung übergeht und der Innenschaft zerstört werden kann.

Das Verbindungselement ist vorzugsweise integral mit dem Innenschaft ausgebildet und außerhalb der Achsmitte angeordnet, sodass durch die mechanische Verbindung zwischen dem Außenschaft und dem Innenschaft durch die Relativbewegung zwischen dem Außenschaft und dem Innenschaft eine Kraft über das Verbindungselement übermittelbar ist. Die Kraft greift außerhalb der Achsmitte an. Durch die Kraft wird der Weg auf der Seite des Verbindungselements verkürzt und der Innenschaft knickt in Richtung des Verbindungselements ab.

Der Handgriff weist in einer bevorzugten Ausführung eine Bremseinrichtung auf, die eine Bremskraft auf den Außenschaft ausübt. Der Betätigungsmechanismus bildet ein Drehrad mit einem Übersetzungsgetriebe, das mit dem Außenschaft zur Übertragung der Schubkraft verbunden ist.

Die Sicherheit des Instruments gegen ungewolltes Bewegen der Schaftspitze beispielsweise bei der Verwendung zusammen mit einem Trokar wird durch die Bremseinrichtung verbessert. Die von der Bremseinrichtung auf den Außenschaft ausgeübte Bremskraft verhindert, dass dieser bei einer Anwendung, beispielsweise beim Einführen durch einen Trokar in proximaler Richtung verschoben wird. Die Bremskraft führt also zu einer Selbsthemmung des Außenschafts, die diesen gegen ungewolltes Verschieben sichert. Damit wird eine ungewollte Bewegung der Schaftspitze vermieden.

Zur Änderung der Winkellage der Schaftspitze ist der Außenschaft durch Betätigen eines Drehrades mit einer Betätigungskraft beaufschlagbar. Diese Kraft wird üblicherweise durch den Finger des Anwenders aufgebracht. Der Widerstand des Drehrades liegt in einem als ergonomisch angenehm empfundenen Bereich. Dadurch kann das Instrument, insbesondere die Abwinkelung des Schafts, leicht und sicher vom Anwender erfolgen. Dazu weist das Instrument bei dieser Ausführung ein Übersetzungsgetriebe auf, das durch den Betätigungsmechanismus gebildet ist und mit dem Außenschaft zur Übertragung der Schubkraft verbunden ist. Das Übersetzungsgetriebe gleicht die von der Bremseinrichtung aufgebrachte Bremskraft aus, so dass das Drehrad bzw. allgemein der Betätigungsmechanismus leicht zu bedienen ist.

Der Außenschaft kann drehfest oder drehbar im Handgriff gelagert sein. Wenn der Außenschaft in Umfangsrichtung drehbar im Handgriff gelagert ist, kann durch eine Drehung des Schafts die Position der abgewinkelten Schaftspitze verändert werden. Somit kann die Applikationsposition bei abgewinkeltem Außenschaft leicht verändert werden.

Es versteht sich, dass das Drehrad in zwei Richtungen (Uhrzeigersinn / Gegenuhrzeigersinn) drehbar ist, sodass der Außenschaft in distaler Richtung und in proximaler Richtung bewegt werden kann.

Zusammengefasst wird bei dieser Ausführung die Sicherheit des Instruments verbessert, weil der Außenschaft gegen ungewolltes Verschieben durch die Bremseinrichtung gesichert ist. Gleichzeitig wird eine leichtgängige Bedienung des Instruments beibehalten, da der Betätigungsmechanismus ein Übersetzungsgetriebe bildet, das die vom Anwender aufgebrachte Fingerkraft in die auf den Außenschaft wirkende Schubkraft umwandelt. Das Übersetzungsgetriebe wirkt dabei wie eine Hebelanordnung, die die Schubkraft im Vergleich zur Fingerkraft erhöht.

Zur weiteren Erhöhung der Hebelkraft kann das Drehrad einen hebelartigen Vorsprung aufweisen, der sich radial ausgehend vom Außenumfang des Drehrades erstreckt und mit dem Finger betätigbar ist.

Vorzugsweise umfasst das Drehrad ein Antriebsrad und wenigstens ein drehfest mit dem Antriebsrad verbundenes Abtriebsrad, das mit dem Außenschaft zur Übertragung der Schubkraft verbunden ist. Der Durchmesser des Abtriebsrades ist kleiner als der Durchmesser des Antriebsrades. Damit wird auf einfache Weise die für eine leichtgängige Betätigung des Außenschafts erforderliche Übersetzung erreicht. Ein weiterer Vorteil dieser Ausführungsform ist die kostengünstige und sichere Konstruktion, die diese Ausführungsform bietet.

Der Betätigungsmechanismus kann einen in Schubrichtung axial beweglichen Schlitten umfassen, der einerseits mit dem Außenschaft und andererseits mit dem Übersetzungsgetriebe verbunden ist. Damit wird eine robuste und einfache Konstruktion geschaffen, die eine sichere Übertragung der vom Anwender aufgebrachten Antriebskraft auf den Außenschaft bewirkt.

Der Schlitten kann wenigstens eine erste Zahnstange aufweisen, die parallel zur Schubrichtung angeordnet ist und mit dem Abtriebsrad kämmt. Diese Ausführung ermöglicht eine einfache und sichere Umwandlung der Drehbewegung des Drehrades in eine lineare Bewegung des Außenschafts.

Für eine verbesserte Kraftübertragung kann der Schlitten eine zweite Zahnstange parallel zur ersten Zahnstange aufweisen, wobei das Antriebsrad zwischen den beiden Zahnstangen angeordnet und mit einem weiteren Abtriebsrad drehfest verbunden ist. Das weitere Abtriebsrad ist mit der zweiten Zahnstange verzahnt.

Vorzugsweise weist der Handgriff eine Halteplatte mit einer Linearführung auf, in der der Schlitten axial beweglich angeordnet ist. Die Linearführung weist wenigstens einen Durchbruch, insbesondere zwei parallele Durchbrüche, für den Schlitten auf. Die Halteplatte ermöglicht einen kompakten Aufbau, der einen geringen Bauraum für die Lagerung des Schlittens erfordert.

Der Bremsmechanismus kann ein Klemmelement, insbesondere einen Klemmring aufweisen, wobei das Klemmelement im Handgriff gehalten ist und den Außenschaft mit der Bremskraft beaufschlagt. Das Klemmelement bildet ein passives Bremsmittel, das einen einfachen und kostengünstigen Aufbau des Instrumentes ermöglicht.

Bei einer bevorzugten Ausführungsform weist der Betätigungsmechanismus eine Arretiereinrichtung auf, mit der der Außenschaft in wenigstens einer Position, insbesondere in einer vollständig ausgefahrenen Position, fixierbar ist. Die Arretiereinrichtung ist besonders für Trokare geeignet, die einen besonders großen Widerstand beim Einführen des Instrumentes bewirken, wie beispielsweise wiederverwendbare Trokare mit Ventilklappe. Die Arretiereinrichtung dient dazu, den Außenschaft zusätzlich zum Bremsmechanismus zu fixieren, so dass größere Axialkräfte vom Außenschaft übertragen werden können, ohne dass dieser relativ zur Elektrode verschoben wird.

Dabei kann die Arretiereinrichtung wenigstens ein erstes Rastmittel umfassen, das am Schlitten angeordnet ist. Ein zweites Rastmittel ist am Handgriff, insbesondere an der Halteplatte angeordnet, das mit dem ersten Rastmittel zur Fixierung des Außenschafts verbindbar ist. Die beiden Rastmittel haben den Vorteil, dass diese einfach herstellbar sind, beispielsweise durch ein Spritzgussverfahren, und gleichzeitig eine sichere Fixierung des Außenschafts ermöglichen.
Bei einer besonders bevorzugten Ausführungsform sind die Elektrode und der Außenschaft relativ zum Handgriff jeweils um ihre Längsachse drehbar angeordnet.

Die Elektrode ist durch eine Schiebehülse geführt, die den Außenschaft und die Elektrode drehfest und axial beweglich verbindet.

Diese Ausführungsform ist für nicht-rotationssymmetrische Elektroden, wie beispielsweise Spatelelektroden geeignet oder kann dazu verwendet werden, bei abgewinkeltem Außenschaft die Applikationsposition durch eine Drehbewegung um die Schaftlängsachse zu verändern. Damit kann auf einfache Weise die Elektrode in Umfangsrichtung ausgerichtet werden. Diese Ausführungsform hat den Vorteil, dass die Drehung der Elektrode auch dann möglich ist, wenn sich das Instrument im Trokar befindet. Die Drehbewegung wird bei dieser Ausführungsform durch den Außenschaft eingeleitet, der über die Schiebehülse mit der Elektrode drehfest verbunden ist. Die Schiebehülse hat zudem die Funktion, die Relativbeweglichkeit zwischen dem Außenschaft und der Elektrode herzustellen. Dazu bildet die Schiebehülse eine drehfeste und axial bewegliche Verbindung zwischen dem Außenschaft und der Elektrode. Da der Außenschaft aus dem Handgriff hervorragt, sind keine zusätzlichen Bauteile erforderlich, um die Elektrode zu drehen. Der Anwender greift einfach den Außenschaft und dreht diesen zusammen mit der Elektrode.

Dabei kann die Schiebhülse zumindest abschnittsweise eine Profilierung auf dem Innenumfang aufweisen, die mit der zumindest abschnittsweise entsprechend profilierten Elektrode zur Übertragung eines Drehmoments formschlüssig in Eingriff ist. Diese Ausführung ist kostengünstig und sicher, da eine entsprechend profilierte Schiebehülse einfach herstellbar ist und durch den Formschluss eine sichere Momentenübertragung erreicht wird.

Ein kostengünstiger und einfacher Aufbau wird vorzugsweise dadurch erreicht, dass die Schiebehülse und der Schlitten drehbeweglich und in axialer Richtung der Schiebehülse zur Übertragung der Schubkraft fest verbunden sind. Der Schlitten weist einen Haltering auf, der die Schiebehülse zumindest teilumfänglich umgibt.

Die Offenbarung wird nachfolgend mit weiteren Einzelheiten und mit Bezug auf die beigefügten schematischen Figuren näher erläutert. Diese zeigen:
- Fig. 1: einen Längsschnitt des Außenschafts nach einem Ausführungsbeispiel;
- Fig. 2: einen Längsschnitt durch die Einbauteile des Außenschafts, insbesondere die Hülse und Elektrode;
- Fig. 3: eine Seitenansicht der starren Schaftabschnitte;
- Fig. 4: einen Längsschnitt durch den Außenschaft eines Instruments nach einem Ausführungsbeispiel im Bereich des abwinkelbaren Bereichs;
- Fig. 5: eine Seitenansicht des Innenschafts des Instruments;
- Fig. 6: eine perspektivische Seitenansicht des Innenschafts des Instruments nach Figur 4 mit Außenschaft;
- Fig. 7: eine perspektivische Seitenansicht des Innenschafts des Instruments nach Figur 5 mit Elektrode;
- Fig. 8: einen Längsschnitt durch den Handgriff des Instruments nach Figur 4;
- Fig. 9: eine perspektivische Ansicht des Instrumentes nach einem Ausführungsbeispiel, bei dem das Gehäuse teilweise entfernt ist;
- Fig. 10: einen Querschnitt des Instrumentes gemäß Fig. 6, wobei das Drehrad weggelassen ist, und
- Fig. 11: einen Längsschnitt des Instruments gemäß Fig. 6 entlang der Mittelachse.

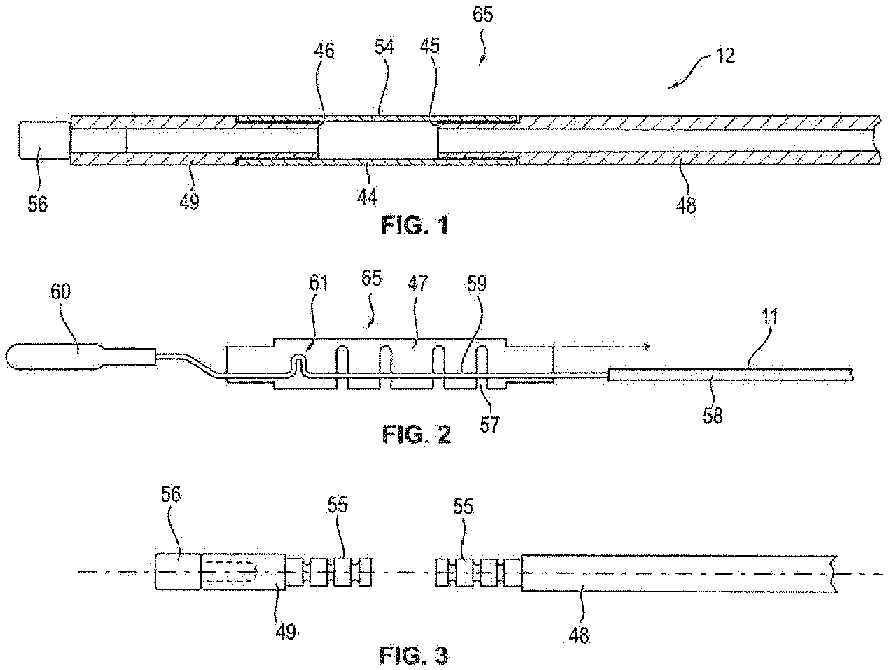

Das Ausführungsbeispiel gemäß den Figuren 1 bis 8 zeigt ein elektrochirurgisches Instrument, das für die Bearbeitung von biologischem Gewebe beispielsweise zur Argonplasma-Koagulation einsetzbar ist. Die Offenbarung ist nicht auf Instrumente für die Argonplasma-Koagulation eingeschränkt, sondern ist allgemein für Instrumente im Bereich der Elektrochirurgie einsetzbar, bei denen eine Schaftspitze aktiv, d.h. vom Handgriff aus, bewegt wird.

Bei der Elektrode 11 des Ausführungsbeispiels kann es sich bspw. um eine Hohlelektrode handeln, die einen Kanal für die Gaszuführung aufweist (APC-Elektrode). Andere Elektroden sind möglich.

Figuren 1 bis 3 zeigen verschiedene Komponenten des Instruments im demontierten Zustand, die im Zusammenhang mit der vom Handgriff 10 aus gesteuerten Abwinklung des Außenschafts 12 im Bereich der Schaftspitze stehen.

Fig. 1 zeigt den Außenschaft 12 des Instruments im distalen Endbereich 65 im Bereich der Spitze ohne die im montierten Zustand im Außenschaft vorgesehenen Einbauteile. Der Außenschaft umfasst im Bereich der Spitze einen ersten und zweiten starren Schaftabschnitt 48, 49, der in Fig. 3 mit weiteren Details gezeigt ist. Zwischen den beiden starren Schaftabschnitten ist der abwinkelbare Bereich 44 angeordnet. Dabei sind ein proximales Ende 45 des Bereichs 44 mit dem ersten, proximalen Schaftabschnitt 48 und ein distales Ende 46 des Bereichs 44 mit dem zweiten, distalen Schaftabschnitt 49 verbunden.

Der abwinkelbare Bereich 44 wirkt wie ein Gelenk und hat die Funktion, eine Relativbewegung zwischen dem ersten und zweiten Schaftabschnitt 48, 49 zu erlauben, so dass ein Winkel zwischen dem ersten Schaftabschnitt 48 und dem zweiten Schaftabschnitt 49 eingestellt werden kann.

Dazu weist der abwinkelbare Bereich einen Schlauch 54 auf, der die beiden starren Schaftabschnitte 48, 49 verbindet. Der Schlauch 54 ist flexibel. Der Schlauch 54 bildet die Außenwand des Außenschafts 12 im Bereich des abwinkelbaren Bereichs 44. Vorteilhafterweise ist der Schlauch 54 als Schrumpfschlauch, insbesondere als Silikon-Schrumpfschlauch ausgeführt, wodurch die Montage erleichtert und eine gasdichte Verbindung zwischen dem Schlauch 54 und den beiden Schaftabschnitten 48, 49 erreicht wird. Andere mechanische Verbindungselemente zwischen den beiden Schaftabschnitten 48, 49 anstelle des Schlauches sind möglich.

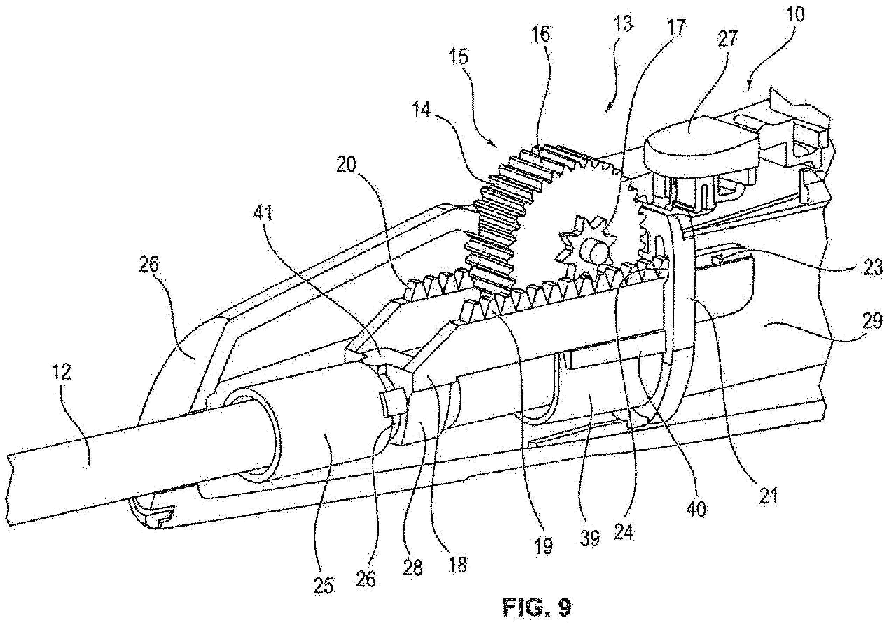

Der proximale erste Schaftabschnitt 48 ist mit dem Handgriff 10 verbunden (siehe Fig. 9).

Zur Verbindung des distalen Endes des ersten Schaftabschnitts 48 mit dem Schlauch 45 kann der erste Schaftabschnitt 48 ein profiliertes Endstück 55 aufweisen, das mit dem Schlauch 54 verbunden ist (Fig. 3). Der zweite distal angeordnete Schaftabschnitt 49 weist am proximalen Ende ebenfalls ein profiliertes Endstück 55 für den Schlauch 54 auf. Am distalen Ende des starren Schaftabschnitts 49 kann eine keramische Endhülse 56 vorgesehen sein, in deren Bereich die Zündelektrode, beispielsweise aus Wolframdraht angeordnet ist.

Anstelle der in Fig. 1 dargestellten einteiligen Ausführung der beiden Schaftabschnitte 48, 49 können diese mehrteilig ausgebildet sein und jeweils ein Stützrohr 50, 51 aufweisen, die zwischen dem Schlauch 54 und einem Schaftrohr angeordnet sind. Die Stützrohre 51, 50 bzw. -hülsen verbessern die Stabilität des Außenschafts und sind aus Metall hergestellt. Die Außenschaftrohre 52, 53, die mit den Stützrohren 51, 50 verbunden sind, sind aus Kunststoff hergestellt.

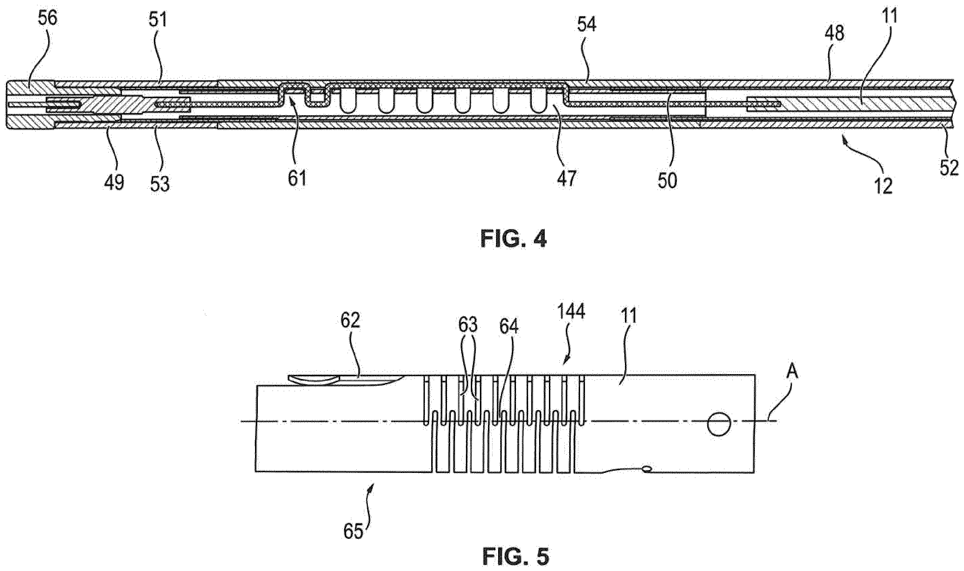

Im montierten Zustand befindet sich das Einbauteil gemäß Fig. 2 im Bereich des abwinkelbaren Bereichs 44, wie in Fig. 4 dargestellt. Das Einbauteil gemäß Fig. 2 umfasst die Elektrode 11 sowie eine Hülse 47, die die Elektrode umgibt. Die Hülse 47 ist aus einem flexiblen Material und weist mehrere Querschlitze 57 auf, die sich quer zur Längserstreckung der Hülse 47 erstrecken. Die Hülse 47 kann zur weiteren Verbesserung der Flexibilität und zur Aufnahme der Elektrode im abgewinkelten Zustand einen Längsschlitz aufweisen (nicht dargestellt).

Die Elektrode 11 bildet drei Abschnitte. Der erste Abschnitt 58 ist starr, beispielsweise aus einem Stahldraht mit einem Durchmesser von ca. 1,5 mm. In der eingebauten Lage befindet sich der erste starre Abschnitt 58 proximal vom abwinkelbaren Bereich 44. Distal des starren Abschnitts 58 ist ein zweiter flexibler Abschnitt 59 vorgesehen, der beispielsweise aus Federstahldraht mit einem Durchmesser von ca. 0,5 mm gebildet ist. Der zweite flexible Abschnitt 59 befindet sich in der Einbaulage im abwinkelbaren Bereich des Schafts und ermöglicht durch seine Flexibilität eine Auslenkung des abwinkelbaren Bereichs 44 bei Betätigung des Betätigungsmechanismus am Handgriff 10.

Dazu ist die Elektrode 11 fixiert. Die Fixierung wird dadurch erreicht, dass die Elektrode 11 mit der Hülse 47 mechanisch verbunden ist und Zug- und Druckkräfte übertragen kann. Die Verbindung der Elektrode 11 mit der Hülse 47 erfolgt an einer Fixierstelle 61, die vom proximalen Ende 45 des abwinkelbaren Bereichs 44 distal beabstandet ist. Im Beispiel gemäß Fig. 2 befindet sich die Fixierstelle 61 in der Einbaulage im Bereich des distalen Endes 46 des abwinkelbaren Bereichs 44. Dadurch wird erreicht, dass beim Verschieben des Außenschafts die gesamte Länge des abwinkelbaren Bereichs 44 zur Auslenkung genutzt werden kann. Es ist möglich, die Fixierstelle in proximaler oder distaler Richtung zu versetzen.

Die Verbindung im Bereich der Fixierstelle 61 kann, wie in Fig. 2 dargestellt, durch eine formschlüssige Verbindung erfolgen. Andere Verbindungsarten sind möglich. Bei dem Beispiel gemäß Fig. 2 ist die Elektrode 11 bogenförmig gekrümmt und greift in die Hülse 47 ein. Mit anderen Worten bildet die Elektrode 11 im Bereich der Fixierstelle 61 eine hakenförmige Verbindung, die mit der Hülse 47 verankert ist. Dadurch wird die Übertragung der Kräfte sicher erreicht.

Distal des zweiten flexiblen Abschnitts 59 der Elektrode 11 ist ein dritter Endabschnitt 60 vorgesehen, in dessen Bereich die Zündelektrode angeordnet ist. Diese kann beispielsweise als Wolframdraht mit einem Durchmesser von ca. 0,5 mm ausgebildet sein.

Nicht dargestellt ist in Fig. 2, dass zusätzlich zur Fixierstelle 61 die Elektrode 11 am proximalen Ende bzw. im proximalen Bereich in einem Handgriff 10 axial angeordnet und ortsfest fixiert ist.

Der Einbauzustand ist in Fig. 4 dargestellt. Darin ist zu sehen, dass die Hülse 47 im Bereich des abwinkelbaren Bereichs 44 angeordnet ist und radial außen vom Schlauch 54 umgeben wird. Ein Unterschied zu dem Beispiel gemäß Fig. 2 ist die Ausbildung der Fixierstelle 61, die in Fig. 4 eine Mehrfachkrümmung zur besseren Verankerung aufweist. Im Unterschied zu dem Stand der Technik ist bei den Ausführungsbeispielen Figuren 1 bis 5 eine Relativbewegung zwischen der Spitze des Außenschafts und der Elektrode zum Exponieren der Elektrode nicht möglich. Damit eignet sich das Instrument gemäß den Figuren 1 bis 5 in erster Linie zum Koagulieren.

Figur 5 zeigt eine weitere Ausführungsform, bei der die Elektrode 11 als Innenschaft 11 ausgebildet ist und in einem distalen Endbereich 65 einen abwinkelbaren Bereich 144 aufweist.

Der abwinkelbare Bereich 144 ist durch Schlitze 63 ausgebildet, die beispielsweise durch Laserschneiden in dem Innenschaft 11 ausgebildet werden. Die Schlitze 63 sind beispielsweise 0,2 mm voneinander beabstandet, wobei die Schlitzbreite zwischen 0,1 und 1,0 mm beträgt.

In der Achsmitte A des Innenschafts 11 sind die Schlitze 63 überlappend angeordnet. Dieser Überlappungsbereich 64 zwischen den gegenüberliegenden Schlitzen 63 beträgt beispielsweise 0,3 bis 0,8 mm, besonders bevorzugt 0,6 mm.

Weiterhin ist in Fig. 5 im distalen Endbereich 65 des Innenschafts 11 ein Verbindungselement 62 abgebildet, wobei das Verbindungselement 62 als Lasche integral mit dem Innenschaft 11 ausgebildet ist. Das Verbindungselement 62 ist vorzugsweise durch Laserschneiden geformt, noch bevorzugter gemeinsam mit den Schlitzen 63. Hierdurch wird eine einfache und kostengünstige Fertigung ermöglicht. Das Verbindungselement 62 ist im abwinkelbaren Bereich 144 oder distal des abwinkelbaren Bereichs 144 mit dem Innenschaft 11 mechanisch verbunden.

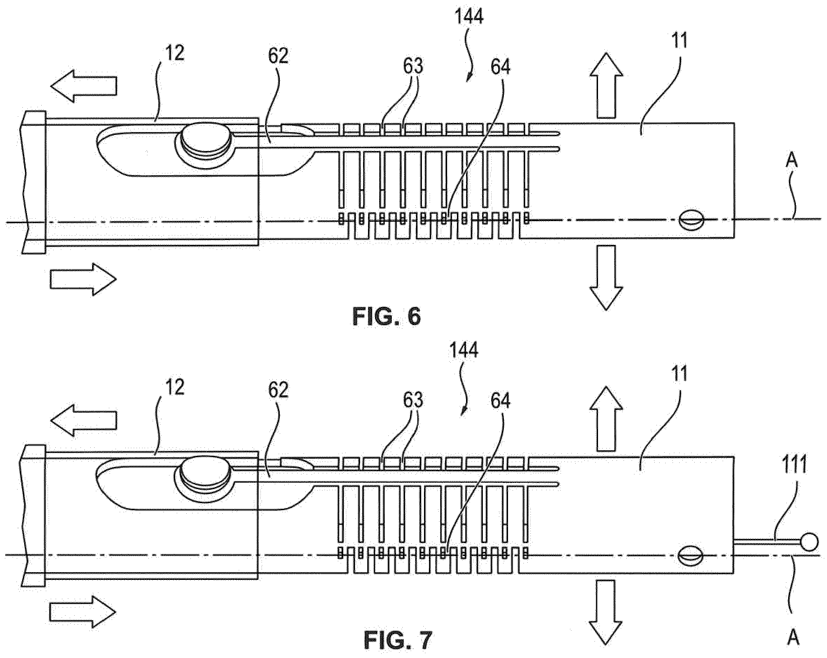

Fig. 6 zeigt die unter Bezug auf Fig. 5 beschriebene Ausbildung des Innenschafts 11, wobei der Innenschaft 11 in Fig. 6 durch das Verbindungselement 62 verbunden mit dem Außenschaft 12 abgebildet ist.

Die Verbindung zwischen dem Innenschaft 11 und dem Außenschaft 12 durch das Verbindungselement 62 ist dergestalt, das bei einer Relativbewegung des Außenschafts 12 gegenüber dem Innenschaft 11 über das Verbindungselement 62 eine Kraft auf den Innenschaft 11 ausgeübt wird derart, das der Innenschaft 11 im abwinkelbaren Bereich 144 seine Ausrichtung ändert und ausgelenkt wird.

Die Abwinklung des Innenschafts 11 geschieht vorzugsweise Senkrecht zu einer Ebene, die durch die Achsmitte A des Innenschafts 11 und die Überlappungsbereiche 64 definiert wird. Ein Abknicken des Innenschafts 11 in dieser Ebene wird durch die Anordnung der Schlitze 63 verhindert.

Fig. 7 zeigt den Fall, in dem eine Elektrode 111 zusätzlich zum Innenschaft 11 vorgesehen ist. In diesem Fall dient also nicht der Innenschaft 11 als Elektrode, wie dies in den unter Bezug auf die Fig. 5 und 6 beschriebenen Ausführungsformen der Fall ist, sondern der Innenschaft 11 umschließt eine Elektrode 111. Die Elektrode 111 kann durch verschiedene Formen gebildet sein. Sie kann in Form einer Nadelelektrode, eine Spatelelektrode oder einer Hülse, als Fortsatz des Innenschafts 11, oder einer anderen Form gebildet sein. Der Innenschaft 11 kann als Kontaktmittel zur Elektrode ausgebildet sein. Alternativ, kann die Elektrode 111 sich auch durch den gesamten Innenschaft 11 bis in den Handgriff 10 erstrecken und innerhalb des Handgriffs 10 mit der elektrischen Versorgung verbunden sein.

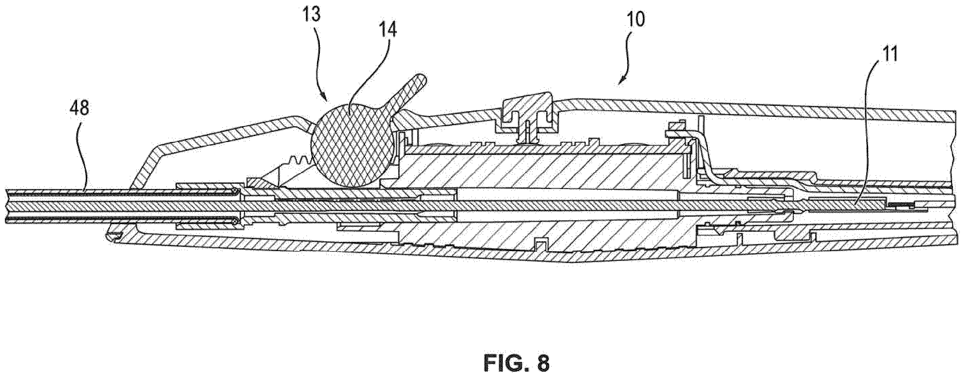

In Figur 8 ist die Anbindung des Betätigungsmechanismus 13 mit dem Drehrad 14 gezeigt. Fig. 8 und Fig. 4 gehören insofern zusammen, als die Weiterführung des Außenschafts 12 aus Fig. 4 in den Handgriff 10 gemäß Fig. 8 gezeigt ist. Die Mechanik zur Umwandlung des Drehmomentes am Handgriff 10 in die translatorische Bewegung des Außenschafts 12 ist in den Figuren 9 bis 11 näher erläutert und wird im Zusammenhang mit dem Ausführungsbeispiel gemäß Figuren 1 bis 4 und 8 offenbart.

Die Elektrode 11 ist im Handgriff 10 ortsfest gelagert. Der Handgriff 10 weist Anschlüsse oder Zuleitungen für die Elektrode auf, die die Stromversorgung und ggf. die Gaszufuhr der Elektrode 11 ermöglichen. Außerdem sind ein oder mehrere Betätigungsmittel, beispielsweise Druckknöpfe 27 am Handgriff vorgesehen. Die Elektrode 11 ist in einem verschiebbaren Außenschaft 12 angeordnet, der über den Handgriff in distaler Richtung vorsteht und im Gehäuse 26 des Handgriffs 10 gehalten ist (s. Fig. 9). Der Außenschaft 12 ist aus einem isolierenden Material hergestellt und umgibt die Elektrode 11 zumindest im Bereich außerhalb des Handgriffes 10.

Der Außenschaft 12 ist relativ zur Elektrode 11 verschiebbar, so dass durch eine Axialbewegung des Außenschafts 12 die Ausrichtung der Spitze des Instruments eingestellt werden kann.

Das Instrument weist eine Bremseinrichtung auf, die den Außenschaft 12 permanent mit einer Bremskraft beaufschlagt und als Verschiebesicherung wirkt. Die Bremskraft oder auch Selbsthemmung des Außenschafts 12 wirkt der Widerstandskraft beim Einführen des Instrumentes in einen Trokar entgegen und verhindert, dass der Außenschaft 12 ungewollt in proximaler Richtung verschoben wird. Der Vorteil der Verschiebesicherung des Außenschafts 12 kommt auch in anderen Situationen zum Tragen, bspw. bei der Präparation.

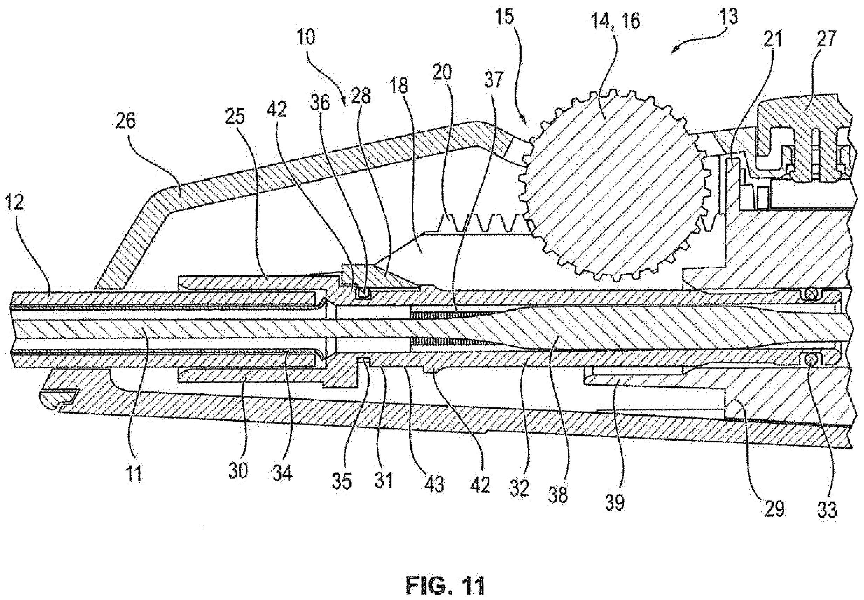

Konkret weist die Bremseinrichtung ein kraftschlüssig wirkendes Klemmelement, beispielsweise in der Form eines Klemmrings 33 auf (Figur 11). Der Klemmring 33 kann ein O-Ring sein. Andere passive Bremsmittel, die der Widerstandskraft im Trokar entgegenwirken, sind möglich. Der Klemmring 33 ist zumindest indirekt mit dem Außenschaft 12 verbunden und überträgt die in den Außenschaft 12 eingeleiteten Axialkräfte in den Handgriff 10, konkret in das Gehäuse 26 des Handgriffs 10. Dazu ist der Außenschaft 12 mit einer Schiebehülse 25 verbunden. Die Schiebehülse 25 und der Außenschaft 12 sind koaxial angeordnet. Die Schiebehülse 25 kann als eine axiale Verlängerung des Außenschafts 12 in den Handgriff 10 hinein verstanden werden. Am distalen Ende der Schiebhülse 25 ist der Klemmring 33 in einer passenden Nut angeordnet derart, dass der Klemmring 33 über den Außenumfang der Schiebehülse 25 vorsteht. Der Klemmring 33 ist im Handgriff 10 abgestützt und erzeugt eine Bremskraft, die einer Kraft, die in Längsrichtung auf den Außenschaft 12 wirkt, beispielsweise der Widerstandskraft im Trokar entgegen wirkt.

Konkret ist die Schiebehülse 25 koaxial in einer Innenhülse 29 angeordnet, die fest mit dem Gehäuse 26, insbesondere durch eine Halteplatte 21, verbunden ist. Der Klemmring 33 drückt gegen den Innenumfang der Innenhülse 29 und erzeugt so eine axial wirkende Bremskraft. Die Innenhülse 29 bildet zugleich die axiale Führung der Schiebehülse 25.

Der Klemmring 33 bzw. allgemein die Bremseinrichtung kann an einer anderen Stelle der Schiebehülse 25 angeordnet sein. Es ist auch möglich, mehr als einen Klemmring 33, beispielsweise zwei Klemmringe zu verwenden.

Um zu vermeiden, dass die Bremseinrichtung die Handhabung des Instrumentes erschwert, bildet der Betätigungsmechanismus 13 ein Übersetzungsgetriebe 15, das mit dem Außenschaft 12 zur Übertragung der Schubkraft verbunden ist.

Der Betätigungsmechanismus 13 weist ein Drehrad 14 auf, das zumindest teilweise aus dem Gehäuse 26 des Handgriffes 10 vorsteht, so dass ein Teilumfang des Drehrades 14 für die Betätigung durch einen Finger zugänglich ist. Die Drehbewegung des Drehrades 14 bewirkt die axiale Verschiebung des Außenschafts 12. Durch Betätigung des Drehrades 14 im Uhrzeigersinn bzw. gegen den Uhrzeigersinn kann der Außenschaft in distaler Richtung vorgeschoben bzw. in proximaler Richtung zurückgezogen werden. Mit anderen Worten kann der Außenschaft 12 hin und her bewegt werden.

Die Funktion des Übersetzungsgetriebes 15 besteht darin, das in das Drehrad 14 eingeleitete Drehmoment so zu wandeln, dass der Außenschaft mit einer erhöhten Schubkraft beaufschlagt wird. Das Übersetzungsgetriebe 15 ist so angepasst, dass die Fingerkraft zur Betätigung des Drehrades 14 kleiner als die Selbsthemmung des Außenschafts 12 ist.

Das Übersetzungsgetriebe 15 umfasst das Drehrad 14, das wiederum ein Antriebsrad 16 und wenigstens ein drehfest mit dem Antriebsrat 16 verbundenes Abtriebsrad 17 aufweist (Figur 9). Das Abtriebsrad 17 ist als Zahnrad ausgebildet, das koaxial mit dem Antriebsrad 16 verbunden ist. Das Antriebsrad 16 kann zur sicheren Bewegung Haltemittel beispielweise in Form einer Riffelung auf dem Außenumfang aufweisen. Dadurch ist das präzise Bewegen des Antriebsrads 16 mittels eines Fingers gewährleistet. Das Drehrad 14 kann als Stufenrad ausgebildet sein, wobei das Antriebsrad 16 und das Abtriebsrad 17 einstückig bzw. integral ausgebildet sind. Alternativ können das Antriebsrad 16 und das Abtriebsrad 17 mechanisch miteinander verbunden sein.

Am Drehrad kann einen Fortsatz als Fingerhebel vorgesehen sein.

Wie in Fig. 9 gut zu erkennen, ist der Durchmesser des Antriebsrades 16 größer als der Durchmesser des Abtriebsrades 17. Konkret ist der Außendurchmesser des Antriebrades 16 um das ca. 2,8-fache größer als der Durchmesser des Abtriebsrades 17. Somit beträgt das Hebelverhältnis ca. 1:2,8. Die erforderliche Fingerkraft ist damit um das 2,8-fache geringer als die Selbsthemmung des Außenschafts 12. Das Hebelverhältnis kann im Bereich von 1:2,6-3,0 liegen, insbesondere im Bereich 1:2,7-2,9.

Ein weiterer Vorteil des Übersetzungsgetriebes besteht darin, dass der Verschiebeweg bzw. das zurückgelegte Bogenmaß am Außendurchmesser des Antriebsrades 16 ebenfalls das 2,8-fache bzw. ein Vielfaches des Verschiebewegs des Außenschafts 12 beträgt. Damit kann eine besonders genaue Einstellung des Schaftwinkels erreicht werden.

Im vorliegenden Ausführungsbeispiel beträgt der Außendurchmesser des Antriebsrades 16 ca. 12,5 mm. Die für die Selbsthemmung des verschiebbaren Außenschafts 12 erforderliche Bremskraft bzw. Klemmkraft beträgt ca. 4 Newton.

Die Umwandlung des vom Drehrad 14 aufgebrachten Drehmomentes in eine translatorische Schubbewegung des Außenschafts 12 wird durch einen Schlitten 18 erreicht, der in proximaler und distaler Richtung axial beweglich ist. Der Schlitten 18 bildet die Verbindung zwischen dem Außenschaft 12 und dem Übersetzungsgetriebe 15. Dazu weist der Schlitten 18 eine erste Zahnstange 19 auf, die parallel zur Schubrichtung des Außenschafts 12 angeordnet ist. Die erste Zahnstange 19 ist mit dem Abtriebsrad 17 verzahnt. Andere Konstruktionen zur Umwandlung der Drehbewegung in eine translatorische Bewegung sind möglich. Im Beispiel gemäß Fig. 9 ist die Zahnstange 19 außenliegend angeordnet. Alternativ kann eine innenliegende Zahnstange vorgesehen sein, die an der Innenseite eines Längsschlitzes ausgebildet ist, der sich parallel zur Mittelachse der Elektrode 11 erstreckt. Das Abtriebsrad 17 ist dann in dem Längsschlitz angeordnet.

Wie Fig. 9 zu entnehmen, weist der Schlitten 18 eine zweite Zahnstange 20 auf, die parallel zur ersten Zahnstange 19 angeordnet ist. Das Antriebsrad 16 ist zwischen den beiden Zahnstangen 19 und 20 angeordnet und mit einem weiteren Abtriebsrad 17 drehfest verbunden. Das weitere Abtriebsrad 17 (nicht dargestellt) kämmt mit der zweiten Zahnstange 20. Der symmetrische Aufbau des Betätigungsmechanismus 13 führt zu einer gleichmäßigen Krafteinleitung sowie zu einer verbesserten Sicherheit des Instruments.

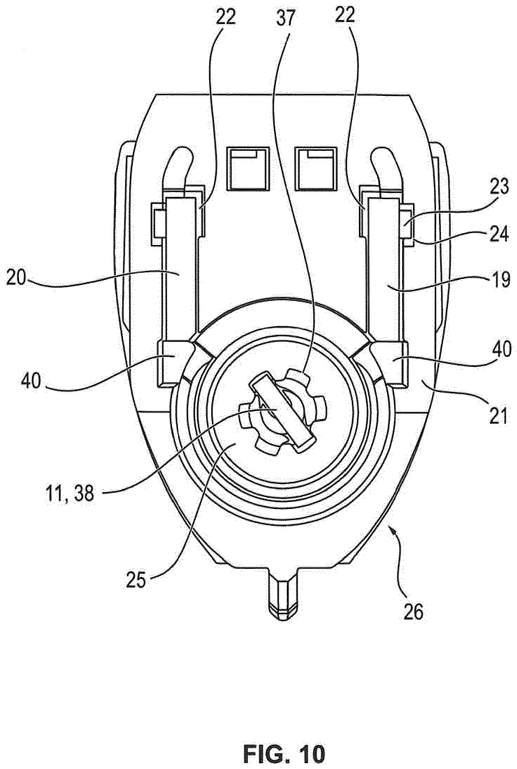

Die beiden Zahnstangen 19, 20 bilden zwei Arme, die sich parallel zur Längsachse der Elektrode 11 und / oder des Außenschafts 12 erstrecken. Die beiden Zahnstangen 19, 20 sind in einer Linearführung angeordnet, die durch die Halteplatte 21 gebildet ist. Die Halteplatte 21 sitzt fest im Gehäuse 26 und weist zwei parallele Durchbrüche 22 für den Schlitten 18 auf (Figur 10). Durch die beiden Durchbrüche 22 sind die Zahnstangen 19, 20 geführt, so dass eine sichere translatorische Bewegung des Schlittens 18 möglich ist. Das Drehrad 14 ist zwischen den beiden Zahnstangen 19, 20 vor der Halteplatte 21 angeordnet, wodurch ein kompakter Aufbau des Handgriffs 10 erreicht wird.

Es ist auch möglich, eine einzige Zahnstange bspw. nur die erste Zahnstange 19 vorzusehen.

Eine weitere Verbesserung der Sicherheit wird durch eine Arretiereinrichtung am Schlitten 18 erreicht. Die Arretiereinrichtung dient dazu, den Außenschaft 12 in einer vorgegebenen Position zu fixieren, insbesondere in der Position in der der Außenschaft 12 und die Elektrode in Längsrichtung gerade, gestreckt angeordnet sind. Die Arretiereinrichtung kann Mittel umfassen, die eine Selbsthemmung des beweglichen Außenschafts 12 sicherstellen. So ist es möglich verschiedene abgewinkelte Positionen des distalen Endes des Instruments zu fixieren.

Im Unterschied zu der Arretiereinrichung, die den 18 Schlitten in einer bestimmten Position festlegt, wirkt die Bremseinrichtung in jeder Position des Schlittens 18, so dass eine stufenlose Verstellung des Außenschafts 12 möglich ist.

Konkret weist die Arretiereinrichtung ein erstes Rastmittel 23 auf, das am proximalen Ende jeweils der ersten und zweiten Zahnstange 19, 20 angeordnet ist. Das erste Rastmittel 23 wirkt im Arretierzustand mit einem zweiten Rastmittel 24 zusammen, das am Handgriff 10 ausgebildet ist. Konkret ist das zweite Rastmittel 24 an der Halteplatte 21 in der Form einer Rastausnehmung ausgebildet. Das erste Rastmittel 23 kann eine entsprechend ausgebildete Rastnase sein, die seitlich an den beiden Zahnstangen 19, 20 angeordnet ist.

Die Arretiereinrichtung verbessert die Gesamtsicherheit des Instruments. Es ist auch möglich, die Arretiereinrichtung unabhängig vom Übersetzungsgetriebe und der Bremseinrichtung zu verwenden, beispielsweise dann, wenn das Instrument ausschließlich mit Trokaren mit sehr hoher Widerstandskraft, wie bspw. bei wiederverwendbaren Trokaren mit Ventilklappe, verwendet werden soll.

Ein weiterer Vorteil des Instruments besteht darin, dass die Elektrode 11 in Umfangsrichtung gedreht bzw. ausgerichtet werden kann, selbst dann, wenn der Außenschaft 12 zumindest teilweise in einem Trokar eingeführt ist. Dazu sind die Elektrode 11 und der Außenschaft 12 jeweils relativ zum Handgriff 10 um ihre Längsachse drehbar angeordnet. Mit anderen Worten können die Elektrode 11 und der Außenschaft 12 zusammen verdreht werden. Dazu ist die Schiebehülse 25 vorgesehen, durch die die Elektrode 11 geführt ist. Die Schiebehülse 25 verbindet den Außenschaft 12 und die Elektrode 11. Dabei handelt es sich um eine drehfeste und axial bewegliche Verbindung. Die Schiebehülse 25 ermöglicht also die Übertragung eines Drehmoments vom Außenschaft 12 auf die Elektrode 11. Gleichzeitig kann die Schiebehülse 25 und somit der mit dieser koaxial bzw. fluchtend verbundene Außenschaft 12 relativ zur Elektrode 11 in axialer Richtung verschoben werden, was die Abwinkelung im Bereich 44 ermöglicht.

Diese Doppelfunktion (Drehmomentübertragung und axiale Verschiebbarkeit) wird dadurch erreicht, dass die Schiebehülse 25 zumindest abschnittsweise eine Profilierung 37 auf dem Innenumfang aufweist. Die Elektrode 11 ist im Bereich der Profilierung 37 entsprechend profiliert und steht zur Übertragung des Drehmoments formschlüssig mit der Schiebehülse 25 in Eingriff. Die formschlüssige Verbindung ist so gestaltet, dass die Schiebehülse 25 entlang der Elektrode 11 sowohl in distaler als auch in proximaler Richtung bewegt werden kann.

Konkret weist die Schiebehülse 25 wenigstens drei Abschnitte auf, nämlich einen distalen Hülsenabschnitt 30, einen mittleren Hülsenabschnitt 31 und einen proximalen Hülsenabschnitt 32. Die Profilierung 37 ist im Bereich des proximalen Hülsenabschnitts 32 ausgebildet. Die Bremseinrichtung, konkret der Klemmring 33 ist am proximalen Ende des proximalen Hülsenabschnittes 32 angeordnet. Die Profilierung 37 erstreckt sich über eine Länge, die in etwa der Länge der beiden Zahnstangen 19, 20 entspricht. Damit wird erreicht, dass die formschlüssige Verbindung zwischen der Elektrode 11 und der Profilierung 37 in jeder Relativlage der Schiebehülse 25 beibehalten wird, so dass die Drehfunktion unabhängig von der jeweiligen Lage des Außenschafts 12 gegeben ist.

Wie in Fig. 10 dargestellt, ist die Profilierung 37 nach Art eines Keilwellenprofils ausgebildet. Dies erhöht die Montagefreundlichkeit, da die entsprechend profilierte Elektrode 11 in die Schiebehülse im Wesentlichen unabhängig von deren Drehposition eingeschoben werden kann. Die Elektrode 11 weist einen Profilabschnitt 38 mit einem rechteckigen Querschnitt auf, wie in Fig. 10 dargestellt. Das proximale und distale Ende des Profilabschnitts 38 der Elektrode 11 verjüngt sich jeweils, wie in Fig. 11 gezeigt. Distal und proximal vom Profilabschnitt 38 weist die Elektrode in herkömmlicher Weise einen im Wesentlichen kreisförmigen Querschnitt auf. Am distalen Ende der Elektrode kann der Querschnitt in einen nicht-rotationssymmetrischen Querschnitt übergehen. Die Elektrode kann bspw. eine Spatelektrode sein.

Der mittlere Hülsenabschnitt 31 weist in distaler und proximaler Richtung jeweils eine Schulter 42 auf. Zwischen den beiden Schultern 42 ist ein Haltebereich 43 ausgebildet, der drehbeweglich mit dem Schlitten 18 verbunden ist. Der Haltebereich 43 bildet eine Ausnehmung zwischen den beiden Schultern 42. In dieser Ausnehmung ist ein Haltering 28 des Schlittens 18 angeordnet. Der Haltering 28 ist teilweise geöffnet und umschließt die Schiebehülse nur teilumfänglich, so dass der Haltering 28 zur Montage einfach auf die Schiebehülse 25 aufgeclipst werden kann. Der Haltering 28 schlägt an den beiden Schulter 42 an, so dass Axialkräfte bzw. die Schubkraft in proximaler und distaler Richtung zum Bewegen des Außenschafts 12 übertragen werden können. Als weitere Sicherheit weist der mittlere Hülsenabschnitt 31 eine Ringnut 35 auf, in der ein Mitnehmer 36 des Halterings 28 angeordnet ist. Der Mitnehmer 36 und die Ringnut 35 sind relativ zueinander drehbeweglich, so dass die Hülse 25 im Haltering 28 frei drehbar ist. Der Mitnehmer 36 überträgt ebenfalls die Schubkraft in beiden axialen Richtungen.

Der Haltering 28 ist zwischen den beiden Zahnstangen 19, 20 an deren distalen Ende angeordnet. Konkret ist eine Traverse 41 vorgesehen, die die distalen Enden der beiden Zahnstangen 19, 20 verbindet, wie in Fig. 9 gezeigt. Die Traverse 41 wiederum ist mit dem Haltering 28 fest verbunden bzw. einstückig ausgebildet. Die Traverse 41 und der Haltering 28 können auch als eine Traverse mit zwei unten angeordneten Haltebacken angesehen werden, die die Schiebehülse 25 teilumfänglich umgeben.

Zwischen der Traverse 41 und der Halteplatte 21 ist ein ausreichender Abstand vorgesehen, so dass der Schlitten 18 am Drehrad 14 vorbei bewegt werden kann, ohne mit dem Drehrad 14 zu kollidieren.

Die Schiebehülse 25 umfasst ferner einen distalen Hülsenabschnitt 30. Der distale Hülsenabschnitt 30 ist drehfest mit dem Außenschaft 12 verbunden. Die Verbindung kann mechanisch erfolgen, beispielsweise durch eine Befestigungshülse 34, die im Außenschaft 12 angeordnet und am proximalen Ende des Außenschafts 12 mit der Schiebehülse 25 vercrimpt ist. Andere Befestigungsmöglichkeiten sind denkbar. Der distale Hülsenabschnitt 30 bildet zusammen mit dem Gehäuse 26 einen axialen Anschlag, der die maximale Auszugsposition des Außenschafts 12 bestimmt.

Zur Unterstützung der Linearführung des Schlittens 18 weist der Handgriff die vorstehend erwähnte Innenhülse 29 auf, die fest mit der Halteplatte 21 verbunden ist. Die Innenhülse 29 ist koaxial zur Elektrode 11 angeordnet und erstreckt sich distal und proximal von der Halteplatte 21, wie in den Figuren 9, 11 dargestellt. Auf der distalen Seite der Halteplatte 21 bildet die Innenhülse 29 einen Hülsenabschnitt 39 mit zwei Führungsstegen 40, die sich parallel zur Mittelachse der Innenhülse 29 erstrecken. Die Führungsstege 40 bilden Auflageflächen für die beiden Zahnstangen 19, 20 und verbessern so die Stabilität der Linearführung.

Wie in Fig. 11 gezeigt, ist ein Teil der Umfangswand der Innenhülse 29 im Bereich des Drehrades 14 entfernt, um Raum für das Drehrad 14 zu schaffen, das bis auf das für die Fingerbetätigung erforderliche Umfangssegment im Gehäuse 26 angeordnet ist, ohne mit der Innenhülse 29 zu kollidieren. Dies trägt zu einem kompakten Aufbau des Handgriffs bei.

Die Drehfunktion des Handgriffes macht diesen für die Positionierung der abgewinkelten Elektrode 11 geeignet, so dass der Handgriff nicht nur besonders sicher und kostengünstig ist, sondern bedienungsfreundlich ist.

Zusammengefasst kombiniert das Instrument drei Funktionen:
Durch den Betätigungsmechanismus wird vom Handgriff aus die Schaftspitze und der dritte Abschnitt der Elektrode abgewinkelt, so dass der Winkel der Schaftspitze und der Elektrode im Gebrauch verändert werden kann. Der Außenschaft und damit die Elektrode sind um deren Längsachse drehbar, so dass die Elektrode ausgerichtet werden kann, was bei nicht-rotationssymmetrischen Elektroden bzw. zur Änderung der Applikationsposition vorteilhaft ist. Die Bremseinrichtung verhindert ungewollte Positionsänderungen der Schaftspitze, bspw. wenn der Außenschaft in einem Trokar bewegt wird.

Das Instrument wird zusätzlich auch im Zusammenhang mit einem Elektrochirurgiegerät, insbesondere für Anwendung am biologischen Gewebe, beispielsweise für die Argonplasma-Koagulation offenbart.

### Bezugszeichenliste

- 10: Handgriff
- 11: Elektrode, Innenschaft
- 12: Außenschaft
- 13: Betätigungsmechanismus
- 14: Drehrad
- 15: Übersetzungsgetriebe
- 16: Antriebsrad
- 17: Abtriebsrad
- 18: Schlitten
- 19: erste Zahnstange
- 20: zweite Zahnstange
- 21: Halteplatte
- 22: Durchbruch
- 23: erstes Rastmittel
- 24: zweites Rastmittel
- 25: Schiebehülse
- 26: Gehäuse
- 27: Druckknopf
- 28: Haltering
- 29: Innenhülse
- 30: distaler Hülsenabschnitt
- 31: mittlerer Hülsenabschnitt
- 32: proximaler Hülsenabschnitts
- 33: Klemmring
- 34: Befestigungshülse
- 35: Ringnut
- 36: Mitnehmer
- 37: Profilierung
- 38: Profilabschnitt
- 39: Hülsenabschnitt
- 40: Führungssteg
- 41: Traverse
- 42: Schulter
- 43: Haltebereich
- 44: abwinkelbarer Bereich
- 45: proximales Ende
- 46: distales Ende
- 47: Hülse
- 48: erster Außenschaftabschnitt
- 49: zweiter Außenschaftabschnitt
- 50: Stützrohr
- 51: Stützrohr
- 52: Außenschaftrohr
- 53: Außenschaftrohr
- 54: Schlauch
- 55: Endstück
- 56: Endhülse
- 57: Querschlitze
- 58: erster Abschnitt der Elektrode
- 59: zweiter Abschnitt der Elektrode
- 60: dritter Abschnitt der Elektrode
- 61: Fixierstelle
- 62: Verbindungselement
- 63: Schlitze
- 64: Überlappungsbereich
- 65: distaler Endbereich
- 111: Elektrode
- 144: abwinkelbarer Bereich

## Patentansprüche

1. Elektrochirurgisches Instrument, für eine Anwendung am biologischen Gewebe, mit
- einem Handgriff (10),
- einem Außenschaft (12), der eine Elektrode umgibt und im Handgriff (10) gehalten ist, wobei die Elektrode aus einem ersten Abschnitt (58), einem flexiblen zweiten Abschnitt (59) und einem dritten Endabschnitt (60) besteht, und
- einem Betätigungsmechanismus (13) am Handgriff (10) zur Bewegung des Außenschafts (12) in axialer Richtung relativ zu dem ersten Abschnitt (58) der Elektrode,
**dadurch gekennzeichnet, dass**
der Außenschaft (12) und die Elektrode (11, 111) in einem distalen Endbereich (65) des Außenschafts (12) mechanisch miteinander verbunden sind derart, dass der Endabschnitt (60) der Elektrode (11, 111), durch die Bewegung des Außenschafts (12) relativ zu dem ersten Abschnitt (58) der Elektrode (11, 111), abwinkelbar ist, wobei der Außenschaft (12) im distalen Endbereich (65) einen abwinkelbaren Bereich (44) aufweist und die Elektrode (11) in diesem Bereich (44) flexibel ausgebildet ist, wobei der abwinkelbare Bereich (44) des Außenschafts (12), durch die Bewegung des Außenschafts (12) relativ zu dem ersten Abschnitt (58) der Elektrode (11), abwinkelbar ist.

2. Instrument nach Anspruch 1,
**dadurch gekennzeichnet, dass**
die Elektrode (11) an einem distalen Ende (46) des abwinkelbaren Bereichs (44) fixiert ist.

3. Instrument nach einem der Ansprüche 1 oder 2,
**dadurch gekennzeichnet, dass**
der abwinkelbare Bereich (44) eine flexible, insbesondere längs- und/oder quergeschlitzte, Hülse (47) aufweist, die die Elektrode (11) umgibt, und vorzugsweise die Elektrode (11) mit der Hülse (47) zur Übertragung von Druck- und Zugkräften verbunden ist.

4. Instrument nach Anspruch 3,
**dadurch gekennzeichnet, dass**
ein proximales Ende (45) der Hülse (47) mit einem ersten starren Schaftabschnitt (48) verbunden ist, und vorzugsweise ein distales Ende (46) der Hülse (47) mit einem zweiten starren Schaftabschnitt (49) verbunden ist.

5. Instrument nach einem der vorhergehenden Ansprüche,
**dadurch gekennzeichnet, dass**
der abwinkelbare Bereich (44) einen Schlauch (54), insbesondere einen Schrumpfschlauch, aufweist, der die Schaftaußenwand im Bereich des abwinkelbaren Bereichs (44) bildet.

6. Instrument nach Anspruch 1,
**dadurch gekennzeichnet, dass**
ein als Elektrode dienender Innenschaft (11) im distalen Endbereich (65) einen abwinkelbaren Bereich (144) aufweist und der Innenschaft (11) in diesem Bereich (144) flexibel ausgebildet ist, wobei der abwinkelbare Bereich (144) des Innenschafts (11) durch die Bewegung des Außenschafts (12) relativ zum Innenschaft (11) abwinkelbar ist.

7. Instrument nach Anspruch 6,
**dadurch gekennzeichnet, dass**
der Innenschaft (11) im distalen Endbereich (65) mit dem Außenschaft (12), vorzugsweise im Bereich der Schaftspitze, durch ein Verbindungselement (62) mechanisch verbunden ist, wobei das Verbindungselement (62) vorzugsweise als Band, Stift, Lasche oder Steg und flexibel sowie vorzugsweise aus Metall ausgebildet ist.

8. Instrument nach einem der Ansprüche 6 oder 7,
**dadurch gekennzeichnet, dass**
der abwinkelbare Bereich (144) des Innenschafts (11) durch gegenüberliegende Schlitze (63) ausgebildet ist, die vorzugsweise jeweils über die Achsmitte des Innenschafts (11) reichen, um einen Überlappungsbereich (64) auszubilden.

9. Instrument nach Anspruch 8,
**dadurch gekennzeichnet, dass**
die Schlitze (63) ungefähr 0,2 mm zueinander beabstandet sind, und die Überlappung der Schlitze (63) bezüglich der Achsmitte vorzugsweise ungefähr 0,6 mm beträgt.

10. Instrument nach Anspruch 8 oder 9,
**dadurch gekennzeichnet, dass**
die Schlitzbreite der Schlitze (63) 0,1 bis 1,0 mm beträgt.

11. Instrument nach einem der vorhergehenden Ansprüche,
**dadurch gekennzeichnet, dass**
der Handgriff (10) eine Bremseinrichtung aufweist, die eine permanente Bremskraft auf den Außenschaft (12) ausübt, und der Betätigungsmechanismus (13) ein Drehrad (14) mit einem Übersetzungsgetriebe (15) bildet, das mit dem Außenschaft (12) zur Übertragung der axialen Kraft verbunden ist

12. Instrument nach Anspruch 10,
**dadurch gekennzeichnet, dass**
das Drehrad (14) ein Antriebsrad (16) und wenigstens ein drehfest mit dem Antriebsrad (16) verbundenes Abtriebsrad (17) umfasst, das mit dem Außenschaft (12) zur Übertragung der Schubkraft verbunden ist, wobei der Durchmesser des Abtriebsrades (17) kleiner als der Durchmesser des Antriebsrades (16) ist und der Betätigungsmechanismus (13) vorzugsweise einen in Schubrichtung axial beweglichen Schlitten (18) umfasst, der einerseits mit dem Außenschaft (12) und andererseits mit dem Übersetzungsgetriebe (15) verbunden ist und der Schlitten (18) noch bevorzugter wenigstens eine erste Zahnstange (19) aufweist, die parallel zur Schubrichtung angeordnet ist und mit dem Abtriebsrad (17) kämmt.

13. Instrument nach einem der vorhergehenden Ansprüche,
**dadurch gekennzeichnet, dass**
die Elektrode (11, 111) und der Außenschaft (12) relativ zum Handgriff (10) jeweils um ihre Längsachse drehbar angeordnet sind, wobei die Elektrode (11, 111) durch eine Schiebehülse (25) geführt ist, die den Außenschaft (12) und die Elektrode (11, 111) drehfest und axial beweglich verbindet und die Schiebehülse (25) vorzugsweise zumindest abschnittsweise eine Profilierung auf dem Innenumfang aufweist, die mit der zumindest abschnittsweise entsprechend profilierten Elektrode (11, 111) zur Übertragung eines Drehmomentes formschlüssig in Eingriff ist und noch bevorzugter die Schiebehülse (25) und der Schlitten (18) drehbeweglich und in axialer Richtung der Schiebehülse (25) zur Übertragung der Schubkraft fest verbunden sind, wobei der Schlitten (18) einen Haltering (28) aufweist, der die Schiebehülse (25) zumindest teilumfänglich umgibt.

14. Elektrochirurgiegerät, für eine Anwendung am biologischen Gewebe, mit einem Instrument nach einem der Ansprüche 1 bis 13.

## Claims

1. Electrosurgical instrument for use on biological tissue, comprising
- a handle (10),
- an outer shaft (12) which surrounds an electrode and is held in the handle (10), wherein the electrode is composed of a first portion (58), a flexible second portion (59) and a third end portion (60), and
- an actuation mechanism (13) on the handle (10) for moving the outer shaft (12) in an axial direction relative to the first portion (58) of the electrode,
**characterized in that**
the outer shaft (12) and the electrode (11, 111) are mechanically connected to each other in a distal end region (65) of the outer shaft (12) in such a way that the end portion (60) of the electrode (11, 111) is bendable by the movement of the outer shaft (12) relative to the first portion (58) of the electrode (11, 111),
wherein the outer shaft (12) has a bendable region (44) in the distal end region (65), and the electrode (11) is flexible in this region (44), wherein the bendable region (44) of the outer shaft (12) is bendable by the movement of the outer shaft (12) relative to the first portion (58) of the electrode (11).

2. Instrument according to Claim 1, **characterized in that** the electrode (11) is fixed at a distal end (46) of the bendable region (44).

3. Instrument according to either of Claims 1 and 2, **characterized in that** the bendable region (44) has a flexible sleeve (47) which in particular is longitudinally and/or transversely slit and which surrounds the electrode (11), and the electrode (11) is preferably connected to the sleeve (47) for the transmission of compressive and tensile forces.

4. Instrument according to Claim 3, **characterized in that** a proximal end (45) of the sleeve (47) is connected to a first rigid shaft portion (48), and a distal end (46) of the sleeve (47) is preferably connected to a second rigid shaft portion (49).

5. Instrument according to one of the preceding claims, **characterized in that** the bendable region (44) has a hose (54), in particular a shrink hose, which forms the outer wall of the shaft in the region of the bendable region (44).

6. Instrument according to Claim 1, **characterized in that** an inner shaft (11) serving as electrode has a bendable region (144) in the distal end region (65), and the inner shaft (11) is flexible in this region (144), wherein the bendable region (144) of the inner shaft (11) is bendable by the movement of the outer shaft (12) relative to the inner shaft (11).

7. Instrument according to Claim 6, **characterized in that** the inner shaft (11) is mechanically connected, in the distal end region (65), to the outer shaft (12), preferably in the region of the shaft tip, by a connection element (62), wherein the connection element (62) is preferably formed as a band, pin, tab or web and is flexible and preferably made of metal.

8. Instrument according to either of Claims 6 and 7, **characterized in that** the bendable region (144) of the inner shaft (11) is formed by mutually opposite slits (63), which preferably each extend across the axial centre of the inner shaft (11), so as to form an overlap region (64).

9. Instrument according to Claim 8, **characterized in that** the slits (63) are spaced apart from one another by approximately 0.2 mm, and the overlap of the slits (63) with respect to the axial centre preferably measures approximately 0.6 mm.

10. Instrument according to Claim 8 or 9, **characterized in that** the slit width of the slits (63) measures 0.1 to 1.0 mm.

11. Instrument according to one of the preceding claims, **characterized in that** the handle (10) has a brake device which exerts a permanent braking force on the outer shaft (12), and the actuation mechanism (13) forms a rotary wheel (14) with a transmission gearing (15) that is connected to the outer shaft (12) in order to transmit the axial force.

12. Instrument according to Claim 10, **characterized in that** the rotary wheel (14) comprises a drive gear (16) and at least one driven gear (17) which is connected to the drive gear (16) for conjoint rotation therewith and which is connected to the outer shaft (12) in order to transmit the shearing force, wherein the diameter of the driven gear (17) is smaller than the diameter of the drive gear (16), and the actuation mechanism (13) preferably comprises a carriage (18) which is axially movable in a shear direction and which is connected on the one hand to the outer shaft (12) and on the other hand to the transmission gearing (15), and the carriage (18) more preferably has at least a first toothed rack (19), which is arranged parallel to the shear direction and meshes with the driven gear (17) .

13. Instrument according to one of the preceding claims, **characterized in that** the electrode (11, 111) and the outer shaft (12) are each arranged to be rotatable relative to the handle (10) about their respective longitudinal axis, wherein the electrode (11, 111) is guided through a sliding sleeve (25) which connects the outer shaft (12) and the electrode (11, 111) in an axially movable manner and for conjoint rotation, and the sliding sleeve (25) has, preferably at least in part, a profiling on the inner circumference, which profiling is in form-fit engagement with the electrode (11, 111), correspondingly profiled at least in part, in order to transmit a torque, and the sliding sleeve (25) and the carriage (18) are more preferably movable in rotation and rigidly connected in the axial direction of the sliding sleeve (25) in order to transmit the shearing force, wherein the carriage (18) has a retaining ring (28) which surrounds the sliding sleeve (25) at least partly about the circumference.

14. Electrosurgical appliance for use on biological tissue, having an instrument according to one of Claims 1 to 13.

## Revendications

1. Instrument électrochirurgical destiné à être utilisé sur des tissus biologiques et comprenant
- une poignée (10),
- une tige extérieure (12) qui entoure une électrode et qui est maintenue dans la poignée (10), l'électrode comprenant une première portion (58), une deuxième portion flexible (59) et une troisième portion d'extrémité (60), et
- un mécanisme d'actionnement (13) placé au niveau de la poignée (10) et destiné à déplacer la tige extérieure (12) dans la direction axiale par rapport à la première portion (58) de l'électrode,
**caractérisé en ce que**
la tige extérieure (12) et l'électrode (11, 111) sont reliées mécaniquement l'une à l'autre dans une région d'extrémité distale (65) de la tige extérieure (12) de telle sorte que la portion d'extrémité (60) de l'électrode (11, 111) peut être pliée par rapport à la première portion (58) de l'électrode (11, 111) par le mouvement de la tige extérieure (12),
la tige extérieure (12) comportant une région pliable (44) dans la région d'extrémité distale (65) et l'électrode (11) étant conçue pour être flexible dans cette région (44), la région pliable (44) de la tige extérieure (12) étant pliable par rapport à la première portion (58) de l'électrode (11) par le mouvement de la tige extérieure (12).

2. Instrument selon la revendication 1,
**caractérisé en ce que**
l'électrode (11) est fixée à une extrémité distale (46) de la région pliable (44).

3. Instrument selon l'une des revendications 1 et 2, **caractérisé en ce que**
la région pliable (44) comporte un manchon flexible, en particulier fendu longitudinalement et/ou transversalement (47), qui entoure l'électrode (11) et de préférence l'électrode (11) est reliée au manchon (47) pour transmettre des forces de pression et de traction.

4. Instrument selon la revendication 3,
**caractérisé en ce que**
une extrémité proximale (45) du manchon (47) est reliée à une première portion de tige rigide (48) et de préférence une extrémité distale (46) du manchon (47) est reliée à une deuxième portion de tige rigide (49).

5. Instrument selon l'une des revendications précédentes,
**caractérisé en ce que**
la région pliable (44) comporte un tuyau (54), en particulier un tuyau rétractable, qui forme la paroi de tige extérieure au niveau de la région pliable (44).

6. Instrument selon la revendication 1,
**caractérisé en ce que**
une tige intérieure (11) servant d'électrode comporte une région pliable (144) dans la région d'extrémité distale (65) et la tige intérieure (11) est conçue pour être flexible dans cette région (144), la région pliable (144) de la tige intérieure (11) pouvant être pliée par rapport à la tige intérieure (11) par le mouvement de la tige extérieure (12).

7. Instrument selon la revendication 6,
**caractérisé en ce que**
la tige intérieure (11) est reliée mécaniquement dans la région d'extrémité distale (65) à la tige extérieure (12), de préférence dans la région de la pointe de tige, par un élément de liaison (62), l'élément de liaison (62) étant de préférence conçu sous la forme d'une bande, d'une tige, d'une languette ou d'une nervure et de manière flexible et de préférence en métal.

8. Instrument selon l'une des revendications 6 et 7, **caractérisé en ce que**
la région pliable (144) de la tige intérieure (11) est formée par des fentes opposées (63) qui s'étendent de préférence chacune sur le centre axial de la tige intérieure (11) afin de former une région de chevauchement (64).

9. Instrument selon la revendication 8,
**caractérisé en ce que**
les fentes (63) sont espacées d'environ 0,2 mm les unes des autres et le chevauchement des fentes (63) par rapport au centre axial est de préférence d'environ 0,6 mm.

10. Instrument selon la revendication 8 ou 9, **caractérisé en ce que**
la largeur des fentes (63) est de 0,1 à 1,0 mm.

11. Instrument selon l'une des revendications précédentes,
**caractérisé en ce que**
la poignée (10) comporte un dispositif de freinage qui exerce une force de freinage permanente sur la tige extérieure (12), et le mécanisme d'actionnement (13) forme une roue rotative (14) avec un réducteur (15) qui est relié à la tige extérieure (12) pour transmettre la force axiale.

12. Instrument selon la revendication 10,
**caractérisé en ce que**
la roue rotative (14) comprend une roue d'entraînement (16) et au moins une roue de sortie (17) qui est reliée solidairement en rotation à la roue d'entraînement (16) et qui est reliée à la tige extérieure (12) pour transmettre la force de poussée, le diamètre de la roue de sortie (17) étant inférieur au diamètre de la roue d'entraînement (16) et le mécanisme d'actionnement (13) comprenant de préférence un coulisseau (18) qui est mobile axialement dans la direction de poussée et qui est relié à la tige extérieure (12) d'une part et au réducteur (15) d'autre part et le coulisseau (18) comportant de manière encore plus préférée au moins une première crémaillère (19) qui est disposée parallèlement à la direction de poussée et qui s'engrène avec la roue de sortie (17).

13. Instrument selon l'une des revendications précédentes,
**caractérisé en ce que**
l'électrode (11, 111) et la tige extérieure (12) sont chacune disposées de manière rotative autour de leur axe longitudinal par rapport à la poignée (10), l'électrode (11, 111) étant guidée par un manchon coulissant (25) reliant la tige extérieure (12) et l'électrode (11, 111) solidairement en rotation et de manière mobile axialement et le manchon coulissant (25) comportant de préférence au moins par endroits un profilage sur la périphérie intérieure qui est en engagement par complémentarité de formes avec l'électrode (11, 111), profilée au moins par endroits de manière correspondante, pour transmettre un couple et, de manière encore plus préférée, le manchon coulissant (25) et le coulisseau (18) étant reliés de manière mobile en rotation et de manière fixe dans la direction axiale du manchon coulissant (25) pour transmettre la force de poussée, le coulisseau (18) comportant une bague de retenue (28) qui entoure au moins partiellement le manchon coulissant (25).

14. Appareil électrochirurgical destiné à être utilisé sur des tissus biologiques et comprenant un instrument selon l'une des revendications 1 à 13.
